# EUROPEAN PATENT SPECIFICATION

(11) **EP 1 964 590 B1**
(45) Date of publication and mention of the grant of the patent: **10.09.2014**
(21) Application number: 06784078.5
(22) Date of filing: 20.07.2006
(51) Int. Cl.: A61N 5/02, H01S 1/02, H01L 33/34

(54) **PANEL FOR TREATING AND REGENERATING TISSUES AND A RADIATOR FOR STIMULATING BASIC BIOCHEMICAL REACTIONS OF AN ORGANISM**
PANEL ZUR BEHANDLUNG UND REGENERATION VON GEWEBEN UND RADIATOR ZUR STIMULIERUNG VON GRUNDLEGENEDEN BIOCHEMISCHEN REAKTIONEN EINES ORGANISMUS
DISPOSITIF DE TRAITEMENT ET DE REGENERATION DES TISSUS ET DIODE ELECTROLUMINESCENTE DESTINEE A STIMULER DES REACTIONS BIOCHIMIQUES DANS UN ORGANISME

(30) Priority: 20.07.2005 RU 2005123060
(43) Date of publication of application: 03.09.2008
(73) Proprietor: Obschestvo S Ogranichennoy Otvetstvennostyu 'Dipolnye Struktury', St. Petersburg, 197376 (RU)
(72) Inventor: BAGRAEV, Nikolai Taimurazovich, St.Petersburg, 195256 (RU); KLYACHKIN, Leonid Efimovich, St.Petersburg, 188620 (RU); MALYARENKO, Anna Mikhailovna, St.Petersburg, 191123 (RU)
(74) Representative: ABG Patentes, S.L.
(86) International application number: PCT/RU2006/000387
(87) International publication number: WO 2007/011267

(56) References cited:
- RU-C1- 2 209 097
- RU-C2- 2 193 424
- RU-C2- 2 193 424
- RU-C2- 2 229 906
- RU-C2- 2 229 906
- US-A- 4 646 743
- US-A1- 2002 060 299
- US-A1- 2004 108 468

## Description

The method for stimulating the basic biochemical reactions of an organism at tissue treatment and regeneration can be used in medicine for curing the diseases that feature disorder of cellular energy metabolism, attenuation of enzymatic processes, disorder of injured tissue regenerative function. This method of cure is used at therapy of tissue burning injuries resulted both from thermal and chemical exposure, as well as from penetrating hard radiation, e.g. y, α, β-radiation, that can result in malignant tumors and leucosis. The method can be used also at parodontosis and periodontitis treatment, during remission following local anaplastic face surgery, and for curing other diseases of the same class.

The known invention 'Method for exposing cells to electromagnetic radiation', patent No. RU 2197303, published 2003.01.27, Int. CI. A61 N5/02, covers exposure to electromagnetic fields generated by the conductor electromagnetic field. The invention offers creation of wider range of electromagnetic radiation that promotes intact cells activity, causes destruction of malignant cells and capsulation on malignant tumors. However, exposure of cells to microwaves can bring to undesirable side effects since frequency range with regulated emissive power is not rigorously defined. This method does not allow for obtaining sufficiently wide range of electromagnetic field influencing living organism cells that would match near-resonance frequency of these cells oscillation.

The known invention 'Method for treatment of injured biological tissues by exposing lesion focus to physical agents', patent No. RU 2226412, published 2004.04.10, Int. CI. A61 N5/02, describes exposure to high-frequency electromagnetic field generated by amplitude-modulated sine signal with carrier frequency in the range of 100 - 1000 kHz and modulation frequency continually changing within the range of 50 - 8000 Hz. The method offers increasing emission power that provides spread of current induced in biological tissue mainly throughout vessels and intertissue fissures, the cells being skirted. This results in improved curative effect due to acceleration of biological tissues regeneration. However, resonant oscillatory circuit does not provide resonance within the entire range of intracellular oscillating processes that covers frequency spacing from unit Hertz to terahertz. During exposure to electromagnetic waves of the suggested frequency, said exposure being limited to shallow penetration of millimeter waves, rays do not reach deeper layers of injured tissue, whereas membranes of both affected and intact cells in near-surface layers will destruct.

The suggested method is considered to be most close to invention 'Method for wave therapy', patent RU No. 2229906, published 2004.06.10, Int. CI. A61 N5/02, A61N5/06, that describes exposure to electromagnetic radiation with concurrent smooth frequency variation in the entire range of actuating signal, said exposure covering frequency range of 30 GHz to 3 THz with radiation power 10⁻¹¹ - 10⁻¹⁰ W. At the same time, influence of rays of said frequencies goes with resonant response of the organism and correction of disturbed homeostasis. However, resonant oscillatory circuit does not provide resonance within the entire range of intracellular oscillating processes. Besides, rays with mentioned frequency fail to reach deeper layers of injured tissue, and required therapeutic effectiveness cannot be obtained.

Patent RU 2 193 424 C2 describes a device for tissue treatment which includes a power supply source connected to optic radiation source having system for controlling spectral light emission wavenlength range, linear polarization and modulation frequency providing direct and backward shift on p-n transition of non-coherent infrared radiator calibrated depending on radiation flow density.

Patent US 4646743 discloses an apparatus for treating animal bodies with radiation of a selected frequency or wavelength for providing therapy in the tissues and the bones of the animal. It makes use of a modulated radiation from an infrared source which might be broadband radiation in a selected range of wavelengths.

In order to stimulate the basic biochemical reactions of organism during tissue treatment and regeneration, essential is to provide photobiochemical reactions at molecular level that would bring cells (their molecules, atoms) into excited state due to externally supplied specified amount of energy with frequency close to resonant oscillatory frequency of cell membranes. Moreover, said intracellular oscillating processes feature a set of intrinsic characteristic frequencies. Besides, affected cells, e.g. malignant cells, as well as cells experienced thermal or chemical burn, feature shift of frequency range with respect to corresponding range of intact cells.

If cells are exposed to radiation in short range of frequencies, even if this provided frequency range is close to resonant oscillatory frequency of any of its membranes, it causes hard 'jutter' of other cell membranes and can lead to their destruction. Since a cell features intrinsic range of resonant frequency, both outer and inner membranes resonance should be provided. At the same time, biologically reasonable addition of energy promotes reaction of main transfer of proteins between cell membranes in both intracellular and intercellular space. This will be followed by destruction of affected cells membranes and their removal with blood flow.

Technical result of the suggested method comprises exposure of biological tissues to radiation in the entire range of terahertz frequencies, with concurrent increasing of the rays penetration that results in photo-stimulation of biochemical reactions due to coincidence of terahertz radiation frequency and resonant oscillatory frequency of intercellular (and intracellular) membranes and protein molecules. Hence, technical result comprises improvement of injured biological tissue healing efficiency by means of terahertz radiation exposure.

Technical result can be achieved by means of exposure to terahertz-ranged radiation, the carrier of the rays being radiation in infrared range of wavelengths. Carrier infrared radiation provides increase of terahertz frequencies penetration into biological tissues, that in turn contributes to improved efficiency of the photo-stimulating biochemical reactions due to coincidence of terahertz radiation frequency and resonant oscillatory frequency of the intercellular (and intracellular) membranes and protein molecules.

Technical result can be achieved as follows. Basic biochemical reactions of organism are being stimulated for biological tissues treatment and regeneration. The method comprises exposure of skin areas to terahertz radiation in the range of 0.02 THz to 8 THz, the carrier of the rays being infrared radiation in the range of 1 µm to 56 µm.

Said method provides achieving of the technical result due to the main adenosine triphosphate aided reactions, which contribute to blood flow promotion, said reactions being as follows:
1. The basic exothermic reaction is transformation of adenosine triphosphate (ATP) into adenosine diphosphate (ADP) in a cell, released energy representing an enhancer for transformation of guanosine diphosphate (GDP) into guanosine triphosphate (GTP). Said reaction, that is responsible for intra- and extracellular protein transportation, runs due to ADP phase enzyme influence. Besides, the described reaction can be promoted by means of exposure to radiation in infrared range of wavelengths. However, efficiency of this reaction photo stimulating is rather low under conditions of continuous infrared radiation. Use of infrared radiation modulated in terahertz range and matched with intracellular oscillating processes frequencies (in resonance) improves photo-stimulated reactions efficiency by orders of magnitude greater. Besides, use of infrared radiation as a terahertz rays carrier significantly improves terahertz radiation penetration into biological tissues.
2. Reaction of oxygen transportation in myoglobin and hemoglobin, that features change of oxygen spin state during its trapping and drift from iron ion. This reaction is also controlled by ADP phase. Besides, this reaction efficiency is improved by infrared radiation. However, its influence efficiency can be low if radiation does not serve a carrier of terahertz rays with frequency corresponding to oscillatory frequency of heme pyridine ring.
3. Excitation of protein alpha helix hydrogen bonds is also initiated by ADP phase influence. Besides, hydrogen bonds excitation is due to infrared radiation as well. Use of infrared radiation modulated in terahertz range and matched with intracellular oscillating processes frequencies (in resonance) improves photo-stimulated reactions efficiency by orders of magnitude greater.

Moreover, alleged technical result can be realized only under condition of increased depth of radiation penetration within specified frequency range.

When energy is accumulated in a cell, soliton mechanism of power exchange between excited protein molecules starts.

In the process of exposure to terahertz radiation with the use of infrared radiation as a carrier, the above processes has been controlled by means of regulating summary (cumulative) average luminosity flux (mW/m²) and duration of exposure.

Clinical results given below are the examples illustrating improved efficiency of injured tissue treatment by means of exposure to terahertz radiation.

### Example 1.

The patient at the age of 28, diagnosis: second-degree burn of chest.

Burnt area was exposed to electromagnetic radiation with frequency 0.048 THz. Infrared radiation with wavelength 1 µm - 56 µm was used as a carrier. Course of treatment comprised daily exposure during 22.5 min, 10 days in total. Radiation exposure was performed with the same emitter.

Blood circulation at the area of the wound was controlled according to the method of intact cutaneous covering rheography at the distance 1-3 mm from the edge of the wound. The following parameters have been considered at rheographic results examination:
1 ) rheographic index (normal value 0.07-0.09),
2) index of vessels tonic contraction (normal value 15-18%),
3) index of vessels peripheral resistance (normal value 70-80%).

Prior to treatment, rheographic parameters made as follows: rheographic index = 0.4, index of vessels tonic contraction = 22, index of vessels peripheral resistance = 91. After treatment, rheographic parameters made as follows: rheographic index = 0.09, index of vessels tonic contraction =16, index of vessels peripheral resistance = 86.

### Example 2.

The patient at the age of 35, diagnosis: first-degree burn of forearm.

Burnt area was exposed to electromagnetic radiation with frequency 7.01 THz. Infrared radiation with wavelength 1 µm - 56 µm was used as a carrier. Course of treatment comprised daily exposure during 22.5 min, 10 days in total. Radiation exposure was performed with the same emitter.

Prior to treatment, rheographic parameters made as follows: rheographic index = 0.03, index of vessels tonic contraction = 23, index of vessels peripheral resistance = 89. After treatment rheographic parameters made as follows: rheographic index = 0.07, index of vessels tonic contraction = 17, index of vessels peripheral resistance = 79.

Basing on the performed clinical testing, improved efficiency of healing tissues injured in the result of thermal action has been confirmed, the results having been controlled by means of rheographic observation of blood circulation in injured tissue.

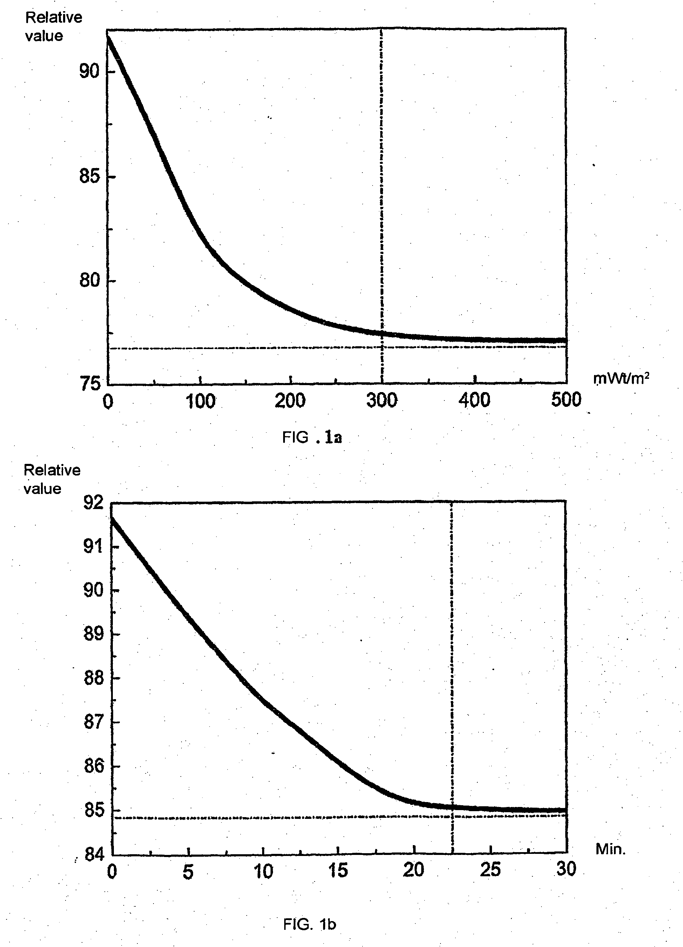

Fig. 1 a, b shows diagrams of decrease of vessels peripheral resistance index down to normal value versus summary average luminosity flux and summary duration of exposure correspondingly.

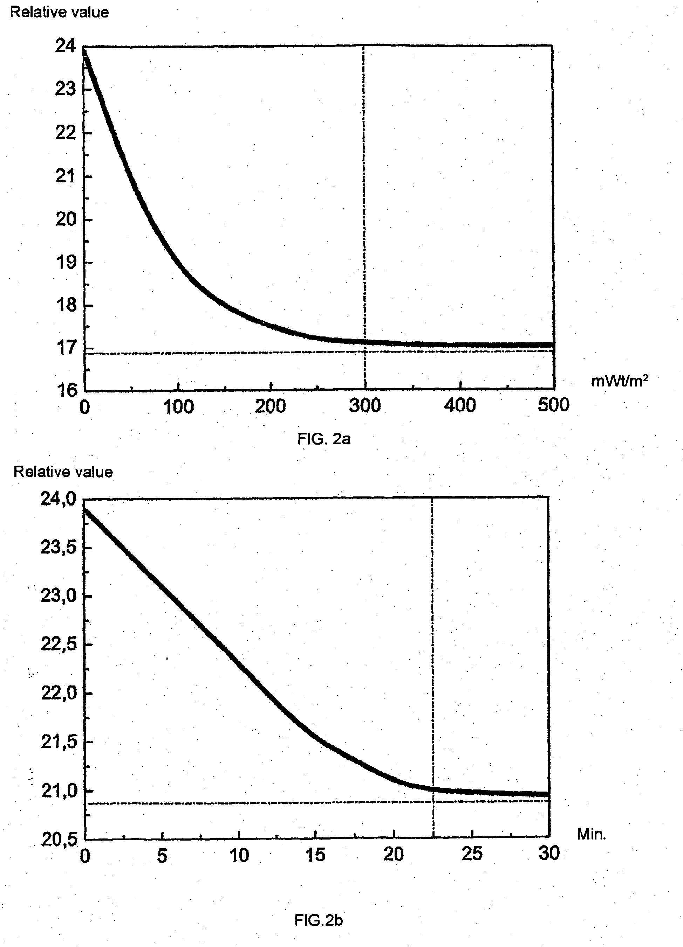

Fig. 2 a, b shows diagrams of decrease of vessels tonic contraction index down to normal value versus summary average luminosity flux and summary duration of exposure correspondingly.

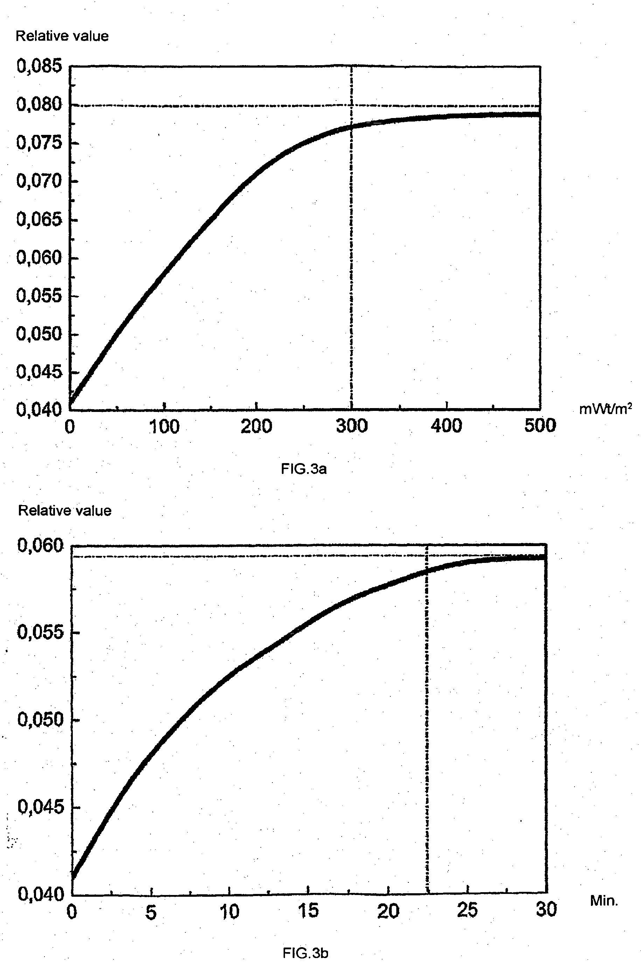

Fig. 3 a, b shows diagrams of increase of rheographic index up to normal value versus summary average luminosity flux and summary duration of exposure correspondingly.

During examination of the results of investigation the following parameters has been considered: 1) rheographic index (normal value 0.07-0.09), 2) index of vessels tonic contraction (normal value 15-18%), and 3) index of vessels peripheral resistance (normal value 70-80%).

Thus, significant improvement of injured tissues healing efficiency due to photo-stimulation of biochemical reactions resulted from coincidence of terahertz radiation frequency and resonant oscillatory frequency of intercellular (and intracellular) membranes and protein molecules, that in turn provides removal of affected cells from organism by intensified blood flow, has been proved. Application of the suggested method resulted in both acceleration of injured tissue healing and absence of complications in the form of inflammatory process and respective vascular disorders.

### A panel for tissue treatment and regeneration

The invention relates to medicine and medical equipment. The panel can be used as a device for considerable acceleration of energy transfer due to extension of injured tissue exposed area in emergency cases, when significant increase of healing surface is necessary (when large area is affected) under conditions of the same duration of a patient exposition and achieving rapid healing response. Otherwise, lingering pain shock can cause irreversible processes, e.g. coma. In particular, the apparatus can be used at shock therapy.

The known invention 'Apparatus for exposing biological objects to EHF-ranged electromagnetic field', patent RU No. 2032430 published 1995.04.10, Int. CI. A61N5/02, describes the apparatus comprising n emitters with fixed electromagnetic radiation frequency, capable of frequency modulation and continuous operation, n making 2 < n < 5. Each of n emitters design provides for their spatial arrangement. EHF-ranged radiation ensures resonance as regards cell resonant frequency spectrum. However, it does not ensure resonance or coincidence with characteristic frequencies within the entire range of intracellular oscillating processes, which can cause complications. Besides, each emitter of the suggested apparatus is discretely tuned to its intrinsic frequency, and the entire apparatus does not provide the required range of radiation frequencies when large affected area is exposed. Moreover, the apparatus does not provide a quantity of excitation sources inside biological tissues; hence, it does not cause bursting effect of intracellular diffusion processes.

The suggested technical decision is considered to be most close to invention 'Apparatus for physical therapy', patent RU No. 2238773, published 2004.10.27, Int. CI. A61 N5/02, which describes the apparatus comprising a power supply unit, M sources of electromagnetic oscillation, M modulators and a power amplifier. Each source of electromagnetic oscillation represents a self-excited oscillator tuned to its intrinsic carrier frequency selected in the range of 100 kHz - 1000 kHz. However, these sources do not provide terahertz frequency of radiation. Besides, since each emitter is discretely tuned to its intrinsic frequency, the apparatus does not provide the required range of radiation frequencies, which would ensure resonance or coincidence with characteristic frequencies within the entire range of intracellular oscillating processes within the entire affected area. Moreover, the apparatus does not provide a quantity of excitation sources inside biological tissues; hence, it does not cause bursting effect of intracellular diffusion processes.

It is common knowledge that the entire spectrum of oscillation frequencies in a cell single parts, e.g. inner and outer cell membranes as well as protein molecules, is defined by intrinsic range of resonant frequencies. At the same time, values of resonant frequencies of a single cell parts differ considerably. However, it is impossible to transfer the required amount of energy of necessary range inside injured tissues since terahertz radiation features low penetrability.

Said circumstance calls for necessity of broader ranges of radiation frequencies with significant depth of their penetration inside tissues. Otherwise, the required healing effect in deep layers of injured tissues will be impossible to achieve, since transfer of biologically reasonable amount of energy that would promote reaction of protein main exchange between cell membranes in inter- and intracellular space, will not be performed. On the other side, significant increase of summary average luminosity flux during exposure can cause further affecting the outer layers of tissue. Besides, the process will get complicated by wide area of lesion. In bad cases, when wide and deep tissue areas are affected, the required healing effect is impossible to achieve with the use of a single emitter of terahertz rays, its carrier being radiation in infrared range of wavelengths.

In emergency cases, when it is essential to maintain duration of a patient exposing to radiation and achieve healing effect within the period at the most of healing minor injured tissue areas, i.e. to achieve bursting intracellular and intercellular diffusion processes responsible for protein transfer, a quantity of sources of cell excitation should be applied. It is impossible just to increase summary average luminosity flux of a single radiation emitter since significant increase of summary average luminosity flux will cause membranes destruction both in affected and intact cells. Besides, since infrared radiation will not penetrate into deep layers of injured tissue within the entire area, this will also decrease curative effect due to impossibility of stimulating the basic biochemical reactions within the entire area of injured tissue.

The more extensive is the injured area, the wider is the range of intrinsic characteristic frequencies spread in a single cell. Therefore, a single radiation source exposure contributes to significant decrease of probability of each cell rapid exciting due to biologically reasonable addition of terahertz radiation, since probability of rapid resonant coincidence with characteristic frequencies within the entire affected area decreases.

In emergency cases, when not only efficient healing is required but also healing process must not prolong, terahertz radiation exposure with the use of a quantity of sources of radiation is applied, the rays' carrier being radiation in infrared range of wavelengths.

All said radiation sources must be tuned in the way that would prevent exceeding permissible value of combined average luminosity flux of all emitters that is defined by biologically reasonable amount of energy added to injured tissue. In order to achieve the required bursting healing effect, it is necessary both to stimulate biochemical reactions of the organism quickly and to provide fast removal of destructed cell membranes from injured area by intensified blood flow. The aforesaid is in particular relative to emergency cases of hazard to the patient's life, when energy transfer throughout the entire organism needed to be intensified considerably. In this case, the required bursting healing effect cannot be achieved without simultaneous transfer of energy from a quantity of the sources of excitation. The mechanism of energy accumulation and transfer by the cells is still understood incompletely. However, it is known that simultaneous several sources of radiation exposure results in soliton effect of protein transfer that promotes healing effect. Constant value of biological rate of protein exchange between cells, as well as mechanism of energy transfer; prevent fast healing with the use of a single radiation emitter.

Hence, technical result comprises improvement of efficiency of injured tissues healing by means of terahertz radiation exposure, said efficiency improvement being provided by means of creation of a quantity of the cell excitation sources at a large area, these excitation sources matching the entire range of cell membranes characteristic frequencies; said technical result further comprising non-linear effect of regenerative influence intensification.

Alleged technical result can be achieved by means of the apparatus described below. A panel for tissue treatment and regeneration comprises n emitters representing silicon light emitting diodes that generate terahertz radiation in the range of 0.02 THz to 8 THz, the carrier of said radiation being infrared radiation in the range of 1 µm to 56 µm and said emitters' combined average luminosity flux being calculated according to medical indications. The panel dimensions are to be defined basing on the required exposure area that provides non-linear effect of regenerative influence intensification at simultaneous activation of light emitting diodes, and the number of light emitting diodes on the panel is defined according to the emitters angular aperture size. In particular, the number of the emitters should be defined with summary average luminosity flux not exceeding 300 mW/m². Dimensions of the panel active zone should be defined basing on the injured area size, as well as on values of power and aperture size of a single source of radiation. A number of segments that incorporate uniformly spaced units (60) of radiation emitters constitute the panel. Besides, light emitting diodes can be activated both in groups and following specified order, e.g. due to exposing hard-to-reach body parts.

Maximum number of emitters should be calculated according to medical indications, the panel dimensions should be defined basing on the required exposure area, and the number of light emitting diodes on the panel should be defined according to the area and depth of tissue injury. When light emitting diodes are activated simultaneously, non-linear effect of the regenerative influence intensification provides both antishock action and effective healing of injured tissues throughout extensive areas without significant prolongation of healing period.

Particular implementation of the panel features 108 emitters, dimensions (W x L) 0.6 x 1.8 m², and 10 cm space between light emitting diodes.
Clinical testing was performed using emitters with power 0.3 W/m².
Summary average luminosity flux made 300mW/m².

### Description of the Figures

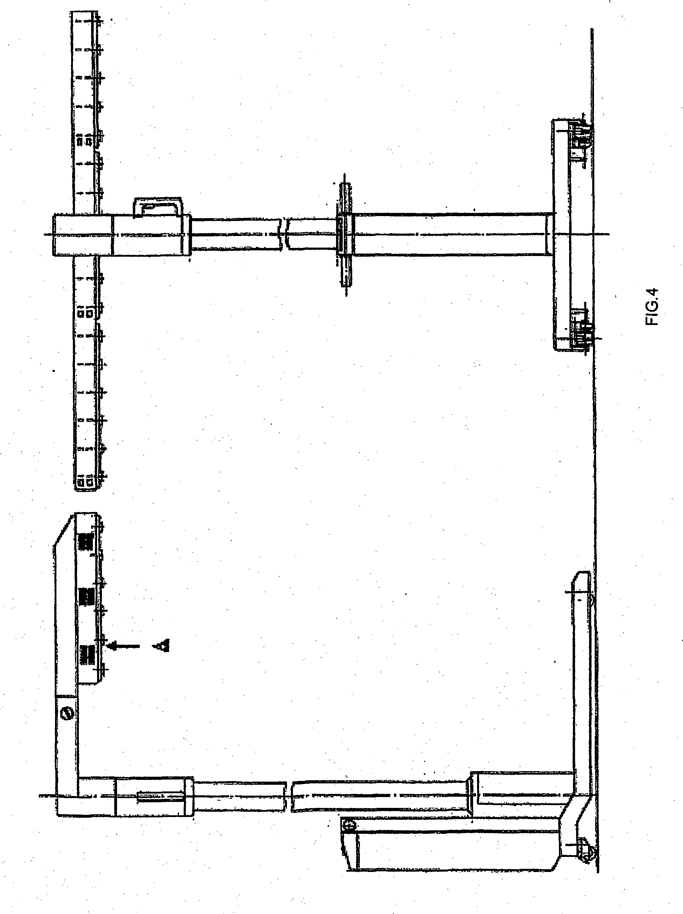

Fig. 4 shows the panel fixed on the rack permitting regulation of its height above an exposed surface, side view and rear view;

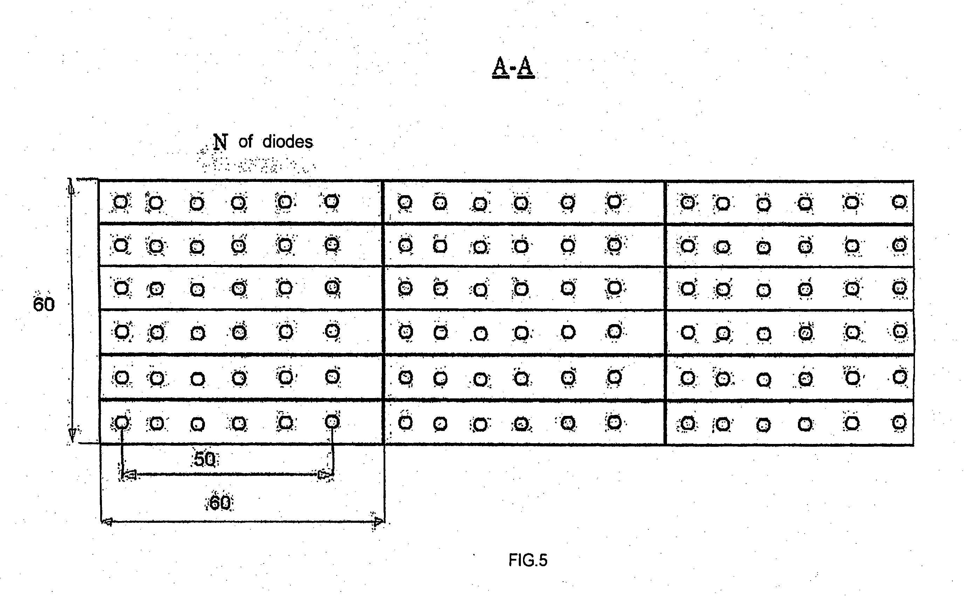

Fig. 5 shows bottom view of the panel constituted by three emitter units (60), each incorporating 36 light emitting diodes.

### Description of principle of apparatus operation.

The panel comprises light emitting diodes with specified radiation parameters. In cases of large area of burns, dimensions of the panel emitting area should be defined according to dimensions and location of injured areas. For instance, three emitter units can be switched on either simultaneously or selectively. Another factor defining the number and selection of activated emitters on the panel is time of distribution and time of excitation transfer from the source of excitation to cells of the organism. Depending on the height of radiation emitter and surface geometry, summary average luminosity flux of all activated radiation sources is defined; this summary average luminosity flux must not exceed maximal permitted value of luminosity flux basing on biological indications. Besides, in order to achieve non-linear effect of regenerative influence intensification, necessary radiation density per 1 cm² of tissue surface should be defined.

Since biological rate of energy accumulation and exchange between cells must not exceed the same at exposing to a single radiation source, concurrent stimulation of basic biological reactions on several areas is required. This significantly increases probability of terahertz radiation resonant coincidence with cell characteristic frequencies, and bursting effect of these reactions stimulation is this way achieved. Thus, in emergency cases of hazard to a patient's life due to pain shock such exposure can provide healing of large areas of injured tissues after 10 sessions, i.e. not longer than at smaller injured areas. This ensures the decrease of hazard to the patient's life.

Basing on the known exposure area of a single radiation emitter, angle aperture, summary average luminosity flux and geometric sequence of light emitting diodes activating can be determined accounting for the height of the radiation emitter.

### Results of clinical testing

### Example 1.

The patient at the age of 39, diagnosis: second- and first-degree multiple burns of the abdomen bottom and both legs.

The whole underpinning was exposed to electromagnetic radiation of frequency 0.025 THz. Infrared radiation with wavelength 1 µm - 56 µm was used as a carrier. Course of treatment comprised daily exposure during 22.5 min, 10 days in total.

Prior to treatment, rheographic parameters made as follows: rheographic index = 0.05, index of vessels tonic contraction = 21, index of vessels peripheral resistance = 90. After treatment, rheographic parameters made as follows: rheographic index = 0.1, index of vessels tonic contraction = 16, index of vessels peripheral resistance = 78.

### Example 2.

The patient at the age of 43, diagnosis: second-degree multiple burns of the back and the right leg.

All injured areas were simultaneously exposed to electromagnetic radiation of frequency 1.2

THz. Infrared radiation with wavelength 1 µm - 56 µm was used as a carrier. Course of treatment comprised daily exposure during 22.5 min, 10 days in total.

Prior to treatment, rheographic parameters made as follows: rheographic index = 0.03, index of vessels tonic contraction = 22, index of vessels peripheral resistance = 89. After treatment, rheographic parameters made as follows: rheographic index = 0.07, index of vessels tonic contraction = 17, index of vessels peripheral resistance = 79.

Thus, supervision of patients with large injured areas showed that the period of healing in the result of simultaneous exposure to several radiation emitters with summary average luminosity flux equal to a single emitter summary average luminosity flux was the same as the period of small injured tissue area healing in the result of exposure to a single radiation emitter. This effect can be explained by improved efficiency of injured tissues healing due to exposure of a quantity of excitation sources to terahertz radiation within the entire range of cell parts characteristic frequencies and non-linear effect of regenerative influence intensification.

### Emitter for tissue treatment and regeneration

The invention relates to semiconductor devices intended for light emitting, in particular to light-emitting diodes with p-n junction as the main source of radiation. The invention can be applied in medical equipment intended for realizing the method for stimulating the basic biochemical reactions of organism at tissue treatment and regeneration.

The known invention 'Apparatus for microwave therapy', patent RU No. 2134597, published 1999.08.20, Int. CI. A61N5/00 A61N5/02, describes an apparatus comprising emitter and current amplifier, placed in a metal thimble with cover, said cover representing dielectric partition, said thimble representing the screen, said emitter featuring frequency of 2.5+0.1 THz and digital noise generator featuring frequency range of 0.2 - 3.0 Hz. The apparatus comprises microresonator analog and permits use of both low frequency and high frequency radiation. However, the apparatus design is complicated, and it does not use quantum-dimensional heterostructures.

The known invention 'Cyan laser diode', patent RU No. 2127478, published 1999.03.10, Int. CI. H01S3/19, describes a diode comprising multiple layers of a semiconductor of Groups II-IV that form p-n junction, the semiconductor substrate with n-type conduction comprising series of consecutive layers as follows: n-type conductive buffer layer, n-type conductive bottom contact layer, n-type conductive bottom enclosure layer, n-type conductive first optical waveguiding layer, active layer with quantum well, p-type conductive second optical waveguiding layer, p-type conductive top enclosure layer, p-type conductive top contact layer. Technical result of the invention comprises creation of laser diode emitting light in spectral region within wavelengths range of 430 nm - 590 nm. However, the diode does not provide high-quality radiation, and infrared carrier is not used.

The known invention 'Semiconductor light amplifier', patent RU No. 2062543, published 1996.06.20, Int. CI. H01S3/18, describes an amplifier based on heterostructure with quantum-dimensional layers of different thickness, located between the pairs of bias voltage supplying electrodes, said heterostructure being produced in the form of alternate or randomly grouped quantum-dimensional layers of different thickness. However, infrared carrier is not used.

The known invention 'Semiconductor electroluminescent light source', patent RU No. 2233013, published 2004.07.20, Int. CI. H01L33/00, describes a light source comprising semiconducting crystal with p-n junction formed inside, said semiconducting crystal generating light flux at direct bias application, said semiconductor light source further comprising bulk or quantum-dimensional heterostructures and/or heterostructures with quantum wells, quantum filaments, quantum dots. This emitter provides radiation in blue-violet spectral region at enhanced luminous efficacy. However, it does not provide the entire spectrum of radiation frequency, from low to high frequencies.

The known invention 'Semiconductor electroluminescent light source', patent application RU No. 2002105900, published 2003.11.20, Int. CI. H01L33/00, describes a light source comprising semiconducting crystal with p-n junction formed inside, said semiconducting crystal generating light flux at direct bias application, said semiconductor light source further comprising luminescent region partially absorbing crystal generated radiation and converting it to radiation with longer dominant wave-length. The crystal comprises bulk or quantum-dimensional heterostructures with active region embedded between wide-band emitters, and/or heterostructures with quantum wells, quantum filaments, and quantum dots. The invention uses polymer matrix providing radiation in blue-violet spectral region at enhanced luminous efficacy. However, production method of the invention is too complicate, and the apparatus is intended for different use as compared to the alleged invention.

The suggested technical decision is considered to be most close to invention 'Apparatus for electromagnetic therapy', patent RU No. 2209097, published 27.07.2003, that describes an apparatus comprising power supply unit and noncoherent infrared emitter with wave length 1 µm - 56 µm, however said emitter does not provide resonance or coincidence with characteristic frequencies within the entire range of intracellular oscillating processes, that can cause complications.

The object comprises providing terahertz radiation in the range of 0.02 THz to 8 THz, the carrier of this radiation being infrared radiation in the range of 1 µm to 56 µm.

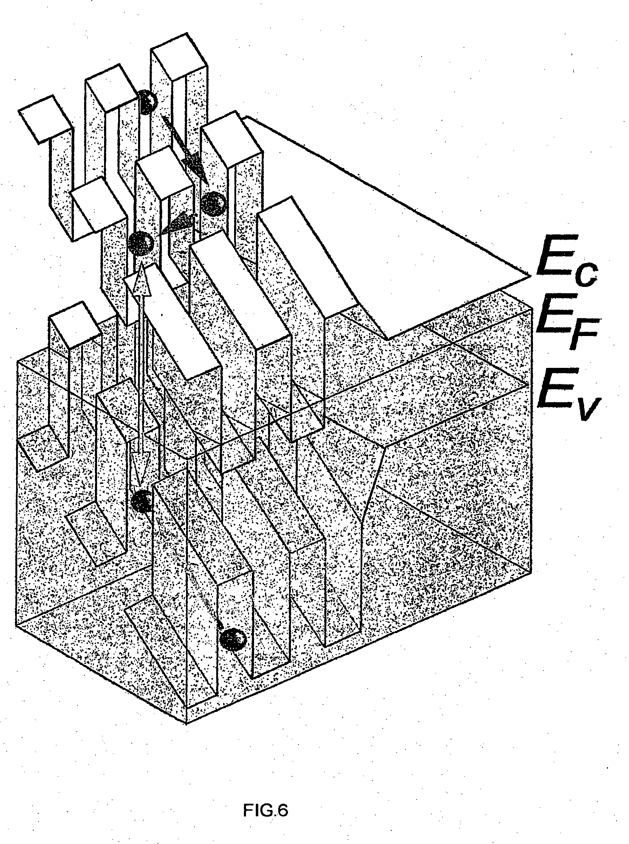

The object can be achieved by means of planar silicon structures that represent a cascade of longitudinal self-ordered silicon quantum wells with p-type conductivity, formed between δ-barriers; said δ-barriers being two-dimensional ferroelectrics (see Fig. 6).

In order to achieve technical result an emitter for tissues treatment and regeneration is suggested, said emitter comprising terahertz radiation source representing semiconductor silicon light emitting diode that generates infrared radiation by means of emitting matrices of planar silicon structures, said infrared radiation being the carrier of terahertz radiation.

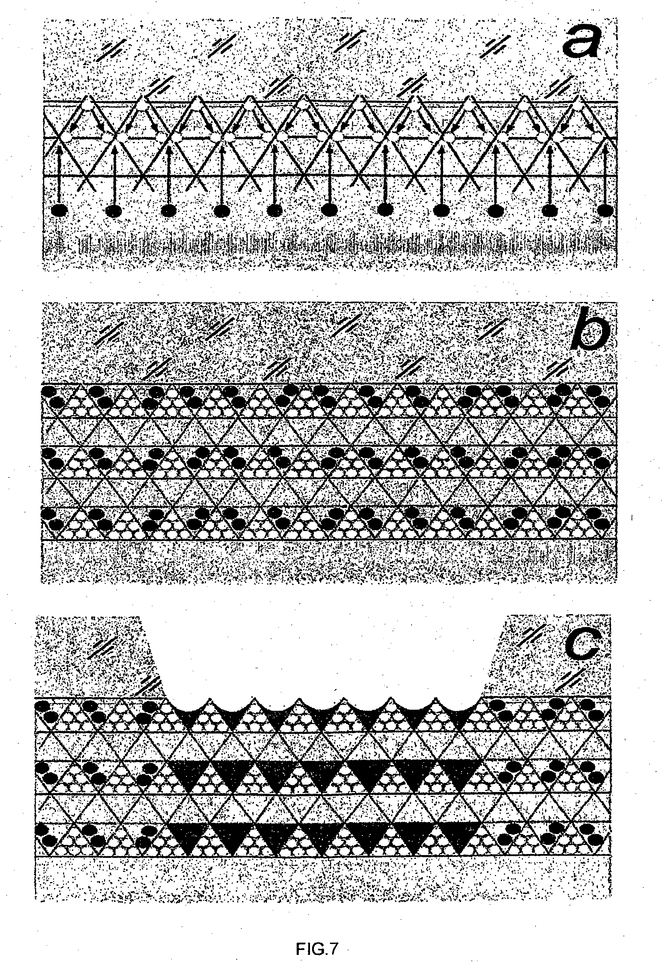

Further, the substrate of emitting matrix represents silicon quantum wells of p-type conductivity, self-ordered inside ultrafine diffusion boron profiles generated in the surface of single-crystalline silicon (100) wafers of n-type conductivity, which provide effect of dimensional quantization and electron-electron interaction upon transfer parameters of single current carriers. The selected wafers are, for instance, of 350-µm thickness, featuring different concentration of fine phosphorus donors. Emitting element is represented by quantum-dimensional p-n junction formed by a cascade of longitudinal silicon self-ordered quantum wells with p-type conductivity, crossed by modulated quantum wire, said quantum-dimensional p-n junction is located inside self-ordered silicon microresonator. Terahertz radiation frequency is provided by means of selecting the geometry of topographic image of ultrafine diffusion boron profiles surface in silicon wafers, said frequency being achieved subject to fluctuations of surface deformation potential which cause forming self-ordered silicon microresonators with distributed feedback in the plane of self-ordered quantum wells. Geometry of p-region is selected, for instance, following Bragg equation, which define its correspondence to terahertz radiation frequency range, and modulation depth is achieved due to the rate of tension, applied both longitudinally and transversely to diffusion profile plane (see Fig. 7). For instance, provided modulated quantum wire has the length of 10 nm and is being formed at the intersection of self-ordered quantum wells.

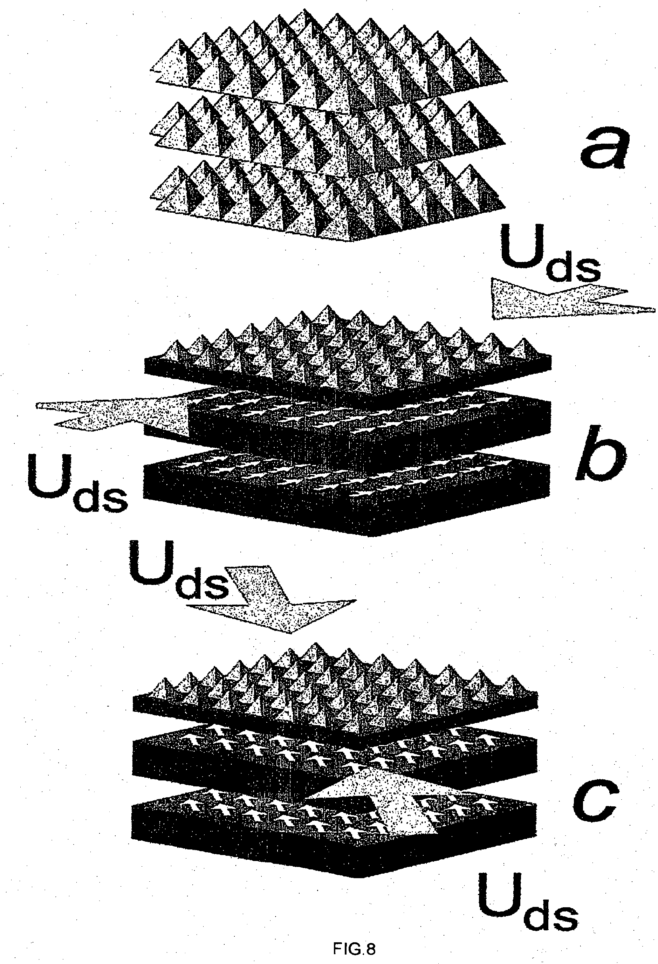

The described light-emitting structures are based on ultrafine p⁺-diffusion profiles with the depth of, for instance, 20 nm, generated in the surface of silicon (100) with n-type conductivity by means of nonequilibrium boron diffusion under conditions of intrinsic defects overflow generation (see Fig. 8).

Emitting silicon matrices represent series of quantum-dimensional p-n junctions created on the surface of a single-crystalline silicon wafer according to planar technology. Display matrices with visible and infrared wavelengths range are the variants of emitting silicon matrices. They comprise a substrate, an emitting element and self-ordered silicon microresonators.

The substrate of emitting matrix represents silicon quantum wells with p-type conductivity, self-ordered inside ultrafine diffusion boron profiles generated in the surface of single-crystalline silicon (100) wafers with n-type conductivity, which provide effect of dimensional quantization and electron-electron interaction upon transfer parameters of single current carriers. Moreover, presence of silicon self-ordered quantum wells inside ultrafine diffusion profiles located between highly doped δ-barriers, is controlled by means of registration of conductivity angular dependences and cyclotron resonance of electrons and holes, e.g. during rotation of magnetic field in the plane perpendicular to the plane of diffusion boron profile in the surface of silicon (100).

Highly doped δ-barriers surrounding a self-ordered quantum well are created by trigonal-doped dipoles B⁺ B⁻ in silicon matrix and feature both properties of ferroelectrics, i.e. metals, and superconductors. During production of display matrices with visible and infrared wavelengths range, δ-barriers with ferroelectric properties are used. Said δ-barriers are formed, for instance, by means of exchange interaction through fine phosphorus donors located in the vicinity of the plane of ultrafine diffusion boron profiles in silicon (100) with n-type conductivity.

Modulated quantum wire represents quasi-one-dimensional structure obtained in the result of intersection of two quantum wells. For instance, quantum wire with the length of 5 µm can be created according to the method of splitted shutter in the plane of a silicon quantum well. (See N.T.Bagraev, A.D.Bouravleuv, W.Gehlhoff, L.E.Klyachkin and A.M.Malyarenko: White light emission from nanostructures embedded in ultra-shallow silicon p-n junction, Proc. of the 26th International Conference on the Physics of Semiconductors (ICPS - 26), Edinburgh,U.K., Physics of Semiconductors 2002, ed. by A.R.Long and J.H. Davies, Inst. of Physics, Conference series, No. 171, G3.3, 2002).

Quantum-dimensional p-n junction representing a cascade of longitudinal silicon self-ordered quantum wells with p-type conductivity, crossed by modulated quantum wire, is used as an emitting element. Said quantum-dimensional p-n junction is located inside self-ordered silicon microresonator.

Point systems (either charged or empty) that can be obtained at complete restriction of current carriers' movement in both quantum wires and wells, and bulk crystals, are used as a quantum dot. They can be obtained by means of electrostatics, of applied metal microcontacts, as well as due to restriction of current carriers' movement in quantum wells and in the process of nanostructures creation (the case in question). In this case, the mode of Coulomb blockade is described by generation of periodic Coulomb peaks resulting from a quantum dot one-electron charge exchange. Coulomb field at quantum dots can feature arbitrary symmetry and be continuous in the view of elementary charge. Filling of relative quantum dots shells depends primarily on the factors like electron-electron interaction and electron spin.

Silicon microresonators with distributed feedback in the plane of silicon self-ordered quantum wells are used as self-ordered silicon microresonators for amplifying radiation defined by interband and intraband transitions between sublevels of dimensional quantization. For instance, microresonators can be formed by means of introducing microdefects into the plane of a self-ordered quantum well. Their geometry is defined by topographic image of ultrafine diffusion boron profiles in silicon, and changes in the result of fluctuations of surface deformation potential due to fractal mechanism of self-ordered point systems forming. Creation of self-ordered microdefects embedded in silicon quantum wells system enables creation of microresonators with distributed feedback and properties that can be identified by means of spectral dependencies of reflection and transmission factors for visible and infrared wavelengths ranges. Self-ordered microresonators significantly decrease light reflection factor in radiation short-wave region, said radiation resulting from direct interband transitions within longitudinal self-ordered quantum wells (d - 2 nm) formed in the plane of ultrafine p⁺-diffusion profile. Microresonators, self-ordering between self-ordered medium-size microdefects, are mostly responsible for decrease of R (λ) within short-wave region (200÷330 nm), whereas presence of larger microdefects promotes effective reflection of light within long-wave region (λ > 330 nm). Spectral dependence of radiation and its intensifying, as well as microresonator size, are interconnected within Bragg relation: *X* = λ/2*n*, where *X* is the microresonator size, λ is wave length, *n* is light refraction index for silicon.

Hence, emitting silicon matrix represents the series of emitting elements, each of said elements being located inside a silicon resonator.

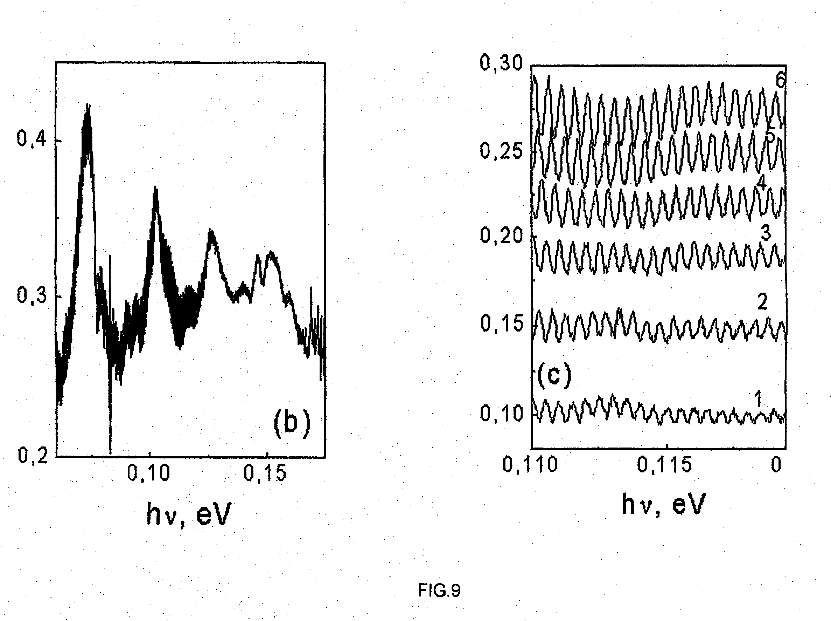

High frequency modulation of infrared radiation can be provided in the form of radiation induced by intraband hole transitions inside a self-ordered quantum well with p-type conductivity, created in the silicon (100) surface with n-type conductivity under conditions of nonequilibrium boron diffusion (see Fig. 9). Spectral dependence of electroluminescence due to emitting transitions between sublevels of heavy and light holes evinces terahertz modulation. Electroluminescence spectra of self-ordered quantum wells, featuring high hole mobility, resulting from emitting transition between sublevels of heavy and light holes evince terahertz and gigahertz modulation due to radiation of boron trigonal dipolar centers B⁺ B⁻, which make the base for δ-barriers confining a quantum well. Modulation depth is increased by introducing microresonators into the plane of a self-ordered quantum well according to Bragg relation: *X = λ*/*2n,* where *X* is the microresonator size, λ is wavelength; *n* is light refraction index for silicon. Modulations generating by boron dipolar centers within gigahertz and terahertz range of 0.02 THz to 8 THz allow use of p⁺ region boundary as a Bragg reflector.

Hence, a single quantum well in single-crystalline silicon surface represents high-power generator of terahertz and gigahertz radiation, boundaries of said quantum well being the emitter of said radiation, whereas the carrier of said radiation being infrared radiation induced by intraband hole transitions. This provides achieving of technical result of the alleged invention.

The figures explain dependencies of radiation frequency spectra with the suggested structure of a light-emitting diode.

Fig. 6 shows planar silicon structure representing a cascade of longitudinal self-ordered quantum wells with p-type conductivity, formed between δ-barriers at the surface of silicon with n-type conductivity.

Fig. 7 Shows the scheme of creating self-ordered quantum wells in the surface of silicon (100) with n-type conductivity under conditions of injecting intrinsic interstitial atoms of silicon (white circles) and vacancies (black circles) during preliminary oxidation (a, b) and subsequent boron diffusion (c) :
a - overflow of intrinsic interstitial atoms of silicon and vacancies generating during preliminary oxidation of silicon (100) surface, these atoms and vacancies featuring corresponding crystallographic orientation along axes [111] and [100].
b - scheme of longitudinal self-ordered quantum wells generating between the layers of microdefects being formed at the step of preliminary oxidation of silicon (100) surface, from intrinsic interstitial atoms of silicon and vacancies.
c - scheme of longitudinal self-ordered quantum wells following subsequent passivation of microdefects under conditions of short-term boron diffusing according to vacancy mechanism (dark areas) following planar silicon technology.

Dimensions of p-type conductive region (longitudinal dimensions of diffusion profile) define terahertz radiation frequency according to Bragg equation.

Fig. 8 shows three-dimensional image of longitudinal self-ordered quantum wells formed between the layers of microdefects (a) that further transform into neutral δ-barriers following passivation under conditions of short-term boron diffusing according to vacancy mechanism ((b), (c)). White arrows show direction of doped boron dipoles ordering inside δ-barriers under conditions of source-sink strain, applied along crystallographic axes [001] (b) and [011] (c).

Fig. 9 shows spectral ranges evincing terahertz (b) and gigahertz (c) modulation of self-ordered silicon quantum wells infrared radiation. (Infrared Fourier spectrometer IFS-115 was used for registration at T=300°K). Current density, A/cm², made as follows: 1 - 50; 2 - 62.5; 3-75; 4-87.5; 5-100; 6-112.5.

Hence, terahertz radiation in the range of 0.02 THz to 8 THz is obtained, the carrier of said radiation being infrared radiation in the range of 1 µm to 56 µm.

## Claims

1. A panel for tissue treatment and regeneration, comprising: n emitters, wherein said emitters are embodied in the form of silicon light emitting diodes generating terahertz radiation in the range of 0.02 THz to 8 THz, a carrier of said radiation 5 being infrared radiation in the range of 1 µm to 56 µm, whereas combined average luminosity flux of said emitters does not exceed 300 mW/m².

2. The panel according to claim 1, further comprised of a number of segments incorporating said emitters into units (60) therein.

## Patentansprüche

1. Feld für Gewebebehandlung und -regeneration, das umfasst: n Emitter, wobei die Emitter in Form von lichtemittierenden Siliciumdioden umgesetzt sind, die eine Terahertz-Strahlung im Bereich von 0,02 THz bis 8 THz erzeugen, einen Träger der Strahlung, bei der es sich um Infrarotstrahlung im Bereich von 1 µm bis 56 µm handelt, wohingegen der kombinierte durchschnittliche Leuchtkraftfluss der Emitter 300 mW/m² nicht überschreitet.

2. Feld nach Anspruch 1, das darüber hinaus aus einer Reihe von Segmenten besteht, die die Emitter in Einheiten (60) darin integrieren.

## Revendications

1. Panneau pour le traitement et la régénération des tissus, comprenant : n émetteurs, lesdits émetteurs étant mis en oeuvre sous la forme de diodes électroluminescentes au silicium générant un rayonnement térahertz dans la plage de 0,02 THz à 8 THz, un support dudit rayonnement étant le rayonnement infrarouge dans la plage de 1 µm à 56 µm, tandis que le flux de luminosité moyen combiné desdits émetteurs ne dépasse pas 300 mW/m².

2. Panneau selon la revendication 1, composé en outre d'un nombre de segments incorporant lesdits émetteurs dans des unités (60).
